# EUROPEAN PATENT SPECIFICATION

(11) **EP 4 579 587 B1**
(45) Date of publication and mention of the grant of the patent: **03.06.2026**
(21) Application number: 24214256.0
(22) Date of filing: 20.11.2024
(51) Int. Cl.: G06T 7/00, G06T 7/11

(54) **METHOD AND SYSTEM FOR PROVIDING REBAR ARRANGEMENT STATE BASED ON COMPUTER VISION**
VERFAHREN UND SYSTEM ZUR BEREITSTELLUNG EINES BEWEHRUNGSANORDNUNGSZUSTANDS AUF BASIS VON COMPUTERVISION
PROCÉDÉ ET SYSTÈME POUR FOURNIR UN ÉTAT D'AGENCEMENT DE BARRES D'ARMATURE SUR LA BASE D'UNE VISION ARTIFICIELLE

(30) Priority: 27.12.2023 KR 20230192844
(43) Date of publication of application: 02.07.2025
(73) Proprietor: Kyungpook National University Industry-Academic Cooperation Foundation, Daegu 41566 (KR)
(72) Inventor: KIM, Bub Ruyr, 41566 Daegu (KR); LEE, Dong Eun, 41566 Daegu (KR)
(74) Representative: AWA Sweden AB

(56) References cited:
- CN-A- 109 448 044
- CN-A- 114 373 123
- XINXING YUAN ET AL: "Automatic evaluation of rebar spacing using LiDAR data", vol. 131, 23 August 2021 (2021-08-23), AMSTERDAM, NL, pages 103890, XP093269470, ISSN: 0926-5805, Retrieved from the Internet <URL:https://www.sciencedirect.com/science/article/pii/S0926580521003411?ref=pdf_download&fr=RR-2&rr=92e91518ee9ddbf0> [retrieved on 20250410], DOI: 10.1016/j.autcon.2021.103890

## Description

### CROSS REFERENCE TO RELATED APPLICATION

The present application claims priority to Korean Patent Application No. 10-2023-0192844, filed on December 27, 2023, the entire contents of which is incorporated herein for all purposes by this reference.

### BACKGROUND OF THE INVENTION

### Field of the Invention

The present invention relates to a method and system for providing a rebar arrangement state based on computer vision.

### [Description of Government-Sponsored Research]

This work is supported by the Korea Agency for Infrastructure Technology Advancement(KAIA) grant funded by the Ministry of Land, Infrastructure and Transport (RS-2023-00251002).

### Description of the Related Art

Structural durability is a crucial factor in ensuring safety in the design and construction of buildings, bridges, and other civil engineering structures, particularly the structural durability significantly differs depending on the arrangement and distribution of rebars.

Specifically, the interval at which rebars are arranged effectively distributes the load of the structure and mitigates stress concentration at specific points, thereby enhancing structural durability. However, when the rebars are not aligned, this can severely affect the load-bearing capacity of the structure, leading to potential hazards.

Accordingly, in conventional methods, the arrangement state of rebars is inspected in various ways. Representatively, a method in which an inspector directly inspects the arrangement state of rebars using a visual aid device may be employed.

Additionally, in recent years, research has been actively conducted on measures for measuring the interval between rebars on the basis of fixing devices, sensor-based devices, images, and computer vision. **In** particular, methods utilizing deep learning models, including convolutional neural networks (CNNs), to detect the arrangement patterns and characteristics of rebars have been developed.

The document by Yuan et al. (2021) "Automatic evaluation of rebar spacing using LiDAR data" discloses a method that relates to the inspection of rebar spacings characterized in that the rebar spacings denote the area between the rebars.

### SUMMARY OF THE INVENTION

The present invention relates to a method and system for providing a rebar arrangement state by detecting spaces formed between rebars in an image captured of a rebar arrangement. The invention is defined by the apended claims.

In addition, the present invention relates to a method and system for providing a rebar arrangement state by classifying the detected spaces in an image according to size.

To solve the aforementioned objects, there is provided a method for providing a rebar arrangement state. The method may include: receiving a rebar image captured of a space in which a plurality of rebars are arranged; inputting the rebar image to a pre-trained space detection model configured to estimate mask regions corresponding to spaces between the plurality of rebars, and obtaining a plurality of mask regions appearing in the rebar image; classifying the plurality of mask regions into a similar group and a unsimilar group on the basis of similarity in size between the plurality of mask regions; and displaying, in the rebar image, the plurality of mask regions corresponding to at least one of the similar group or the unsimilar group to provide an arrangement state of the plurality of rebars.

**In** addition, there is a system for providing a rebar arrangement state, according to the present invention. The system may include: a storage unit storing a rebar image captured of a space in which a plurality of rebars are arranged; and a controller configured to input the rebar image to a pre-trained space detection model configured to estimate mask regions corresponding to spaces between the plurality of rebars, and to obtain a plurality of mask regions appearing in the rebar image, in which the controller may classify the plurality of mask regions into a similar group and a unsimilar group on the basis of similarity in size between the plurality of mask regions and display the plurality of mask regions corresponding to at least one of the similar group or the unsimilar group in the rebar image to provide an arrangement state of the plurality of rebars.

In addition, there is provided a program stored on a computer-readable recording medium, and executed by one or more processes in an electronic device, according to the present invention. The program may include instructions to allow the program to perform: receiving a rebar image captured of a space in which a plurality of rebars are arranged; inputting the rebar image to a pre-trained space detection model configured to estimate mask regions corresponding to spaces between the plurality of rebars, and obtaining a plurality of mask regions appearing in the rebar image; classifying the plurality of mask regions into a similar group and a unsimilar group on the basis of similarity in size between the plurality of mask regions; and displaying, in the rebar image, the plurality of mask regions corresponding to at least one of the similar group or the unsimilar group to provide an arrangement state of the plurality of rebars.

According to various embodiments of the present invention, the system and method for providing a rebar arrangement state can detect spaces formed between rebars in an image captured of arranged rebars, and classify the detected spaces according to size, thereby efficiently verifying the structural arrangement of rebar intervals.

To this end, according to various embodiments of the present invention, the system and method for providing a rebar arrangement state can augment the training rebar image in various ways, enabling more accurate and detailed detection of the spaces formed between rebars in the rebar image.

In particular, according to various embodiments of the present invention, the system and method for providing a rebar arrangement state can repeatedly perform the process of classifying the spaces formed between rebars according to size multiple times, thereby enabling more accurate detection of regions where errors have occurred in the structural arrangement of rebar intervals.

### BRIEF DESCRIPTION OF THE DRAWINGS

FIG. 1 illustrates an embodiment of a method of providing a rebar arrangement state according to the present invention.
FIGS. 2 and 3 illustrate an embodiment of detecting a mask region in a rebar image.
FIGS. 4 and 5 illustrate an embodiment of classifying a mask region.
FIG. 6 illustrates a system for providing a rebar arrangement state according to the present invention.
FIG. 7 is a flowchart illustrating a method of providing a rebar arrangement state according to the present invention.
FIG. 8 illustrates an embodiment of detecting a mask region in a rebar image.
FIGS. 9 to 11 illustrate an embodiment of classifying a mask region.
FIG. 12 illustrates an embodiment of providing a rebar arrangement state.

### DETAILED DESCRIPTION OF THE INVENTION

Hereinafter, exemplary embodiments disclosed in the present specification will be described in detail with reference to the accompanying drawings. The same or similar constituent elements are assigned with the same reference numerals regardless of reference numerals, and the repetitive description thereof will be omitted. The suffixes "module", "unit", "part", and "portion" used to describe constituent elements in the following description are used together or interchangeably in order to facilitate the description, but the suffixes themselves do not have distinguishable meanings or functions. In addition, in the description of the exemplary embodiment disclosed in the present specification, the specific descriptions of publicly known related technologies will be omitted when it is determined that the specific descriptions may obscure the subject matter of the exemplary embodiment disclosed in the present specification.

The terms including ordinal numbers such as "first," "second," and the like may be used to describe various constituent elements, but the constituent elements are not limited by the terms. These terms are used only to distinguish one constituent element from another constituent element.

When one constituent element is described as being "coupled" or "connected" to another constituent element, it should be understood that one constituent element can be coupled or connected directly to another constituent element, and an intervening constituent element can also be present between the constituent elements. When one constituent element is described as being "coupled directly to" or "connected directly to" another constituent element, it should be understood that no intervening constituent element exists between the constituent elements.

Singular expressions include plural expressions unless clearly described as different meanings in the context.

In the present application, it should be understood that terms "including" and "having" are intended to designate the existence of characteristics, numbers, steps, operations, constituent elements, and components described in the specification or a combination thereof, and do not exclude a possibility of the existence or addition of one or more other characteristics, numbers, steps, operations, constituent elements, and components, or a combination thereof in advance.

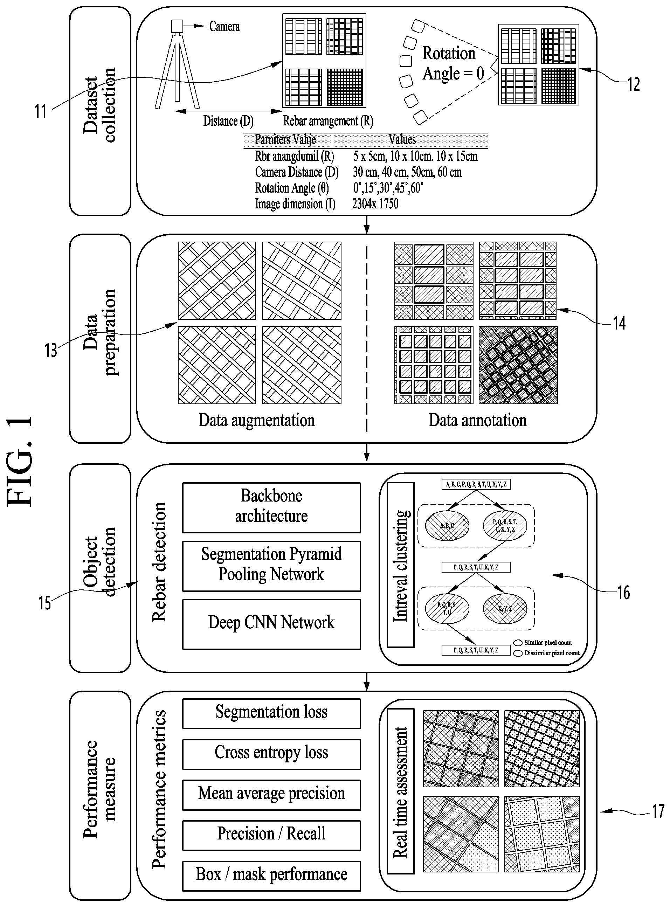

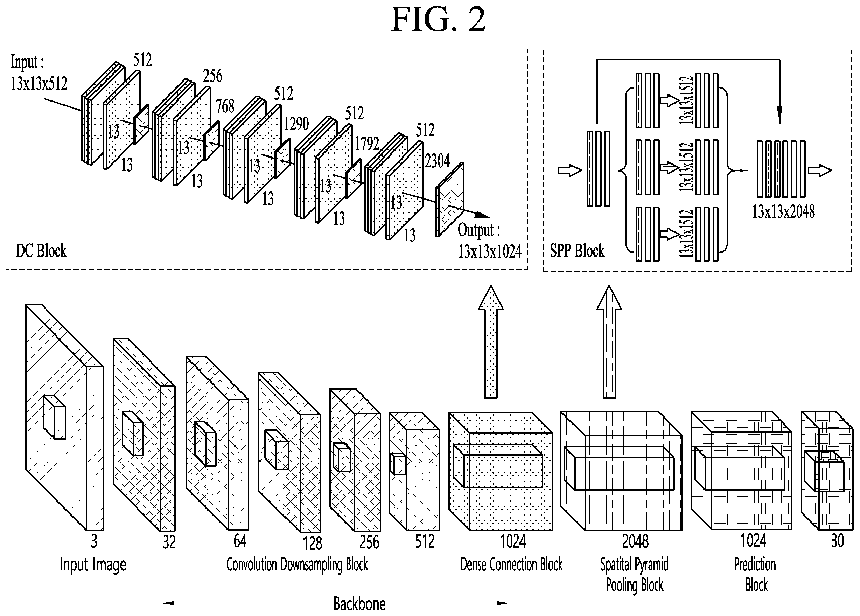

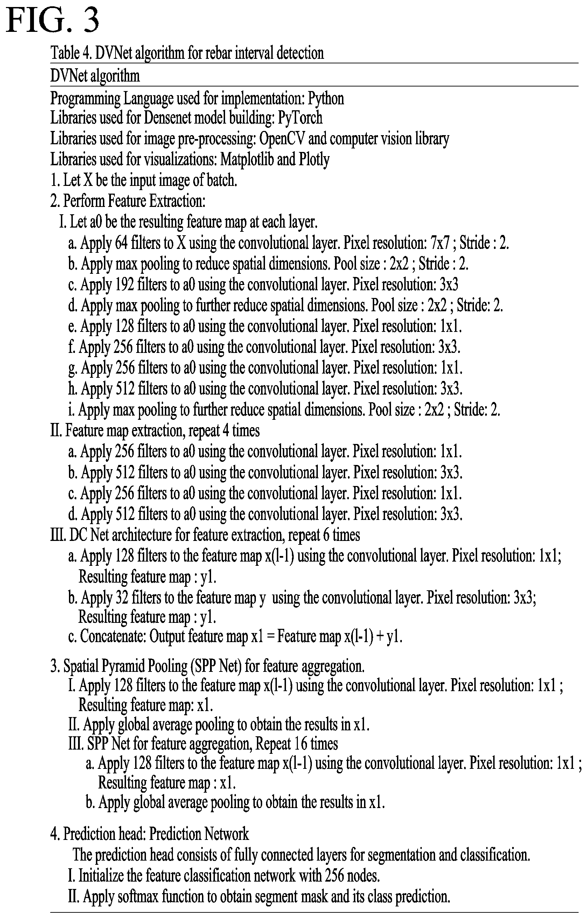

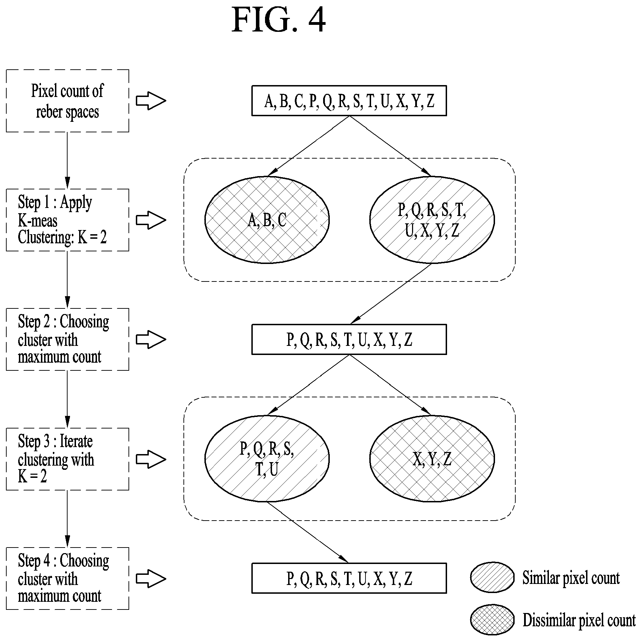

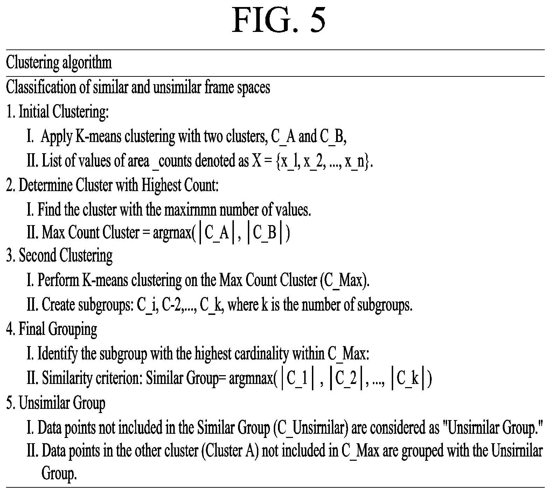

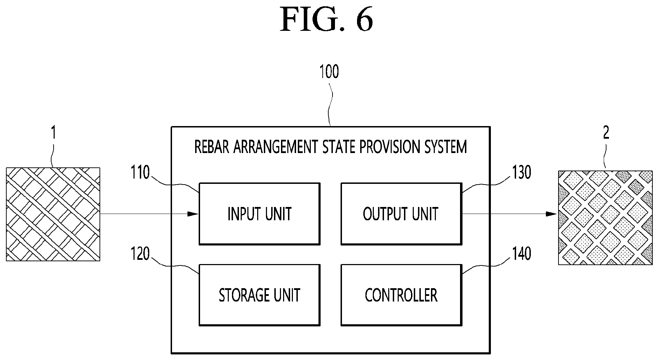

FIG. 1 illustrates an embodiment of a method of providing a rebar arrangement state according to the present invention. FIGS. 2 and 3 illustrate an embodiment of detecting a mask region in a rebar image. FIGS. 4 and 5 illustrate an embodiment of classifying a mask region. FIG. 6 illustrates a system for providing a rebar arrangement state according to the present invention.

With reference to FIG. 1, a system 100 for providing a rebar arrangement state according to the present invention may input a rebar image to a pre-trained space detection model to estimate a mask region for the rebar image.

Here, the rebar image is an image captured of a space in which a plurality of rebars are arranged, and may be an image captured by an RGB camera or a black-and-white camera, etc.

In this case, the plurality of rebars may be arranged to intersect each other along different axes. That is, the rebar image may be an image captured of a space in which a plurality of rebars are arranged to intersect each other.

In addition, the mask region may be a space formed by a plurality of rebars arranged to intersect each other. For example, the mask region may be a rectangular space formed by two rebars arranged adjacent to each other along a first axis and two rebars arranged adjacent to each other along a second axis, in a rebar image captured of a plurality of rebars arranged along the first and second axes to intersect each other perpendicularly.

In this case, the mask region may be implemented in various forms, such as a diamond form or a circular form, depending on an angle at which the plurality of rebars intersect and a shape of each of the plurality of rebars.

The space detection model may be an artificial neural network trained to estimate a mask region in a rebar image. For example, the space detection model may be an artificial neural network implemented based on a deep neural network (DNN) and a convolutional neural network (CNN).

To this end, the space detection model may be trained according to a space detection model training method, which, according to an embodiment, may be performed by the system 100 for providing a rebar arrangement state, or may be performed by a separate training system (or device).

In this case, the training system may store a training rebar image and generate a plurality of training rebar images by augmenting the training rebar image.

Specifically, the training system may store a rebar image captured as a training rebar image by arranging a capturing device (e.g., a camera) at different positions with respect to a space in which a plurality of rebars are arranged.

For example, the training system may store a plurality of training rebar images 11 captured at positions spaced apart by different distances with respect to a space in which a plurality of rebars are arranged.

For another example, the training system may store a plurality of training rebar images 12 captured at positions spaced apart by a predetermined distance at different angles with respect to a space in which a plurality of rebars are arranged.

For another example, the training system may store a plurality of training rebar images captured under different lighting states with respect to a space in which a plurality of rebars are arranged.

For another example, the training system may store a plurality of training rebar images captured of a space in which a plurality of rebars with different dimensions are arranged, or captured of a space in which a plurality of rebars are arranged at different intervals.

Further, the training system may generate a plurality of training rebar images 13 augmented into different shapes by editing a training rebar image.

For example, the training system may generate a flipped training rebar image by editing a training rebar image to be horizontally flipped. Therefore, the training system may train the space detection model so that mask regions existing in various directions and positions are estimated.

For another example, the training system may generate a rotated training rebar image by editing a training rebar image to be rotated at a predetermined angle (e.g., 45 degrees or 135 degrees). Therefore, the training system may train the space detection model so that mask regions are accurately estimated in a rebar image for a wider range of directions.

For another example, the training system may generate a contrast-adjusted training rebar image by editing a training rebar image so that its contrast is adjusted. To this end, the training system may calculate maximum pixel intensity and minimum pixel intensity for a plurality of pixels belonging to a training rebar image and normalize the values of the plurality of pixels on the basis of the calculation results, thereby enhancing the visibility and sharpness of the objects corresponding to the plurality of rebars in the training rebar image. Therefore, the training system may mitigate the effects of noise and artifacts included in the rebar image and train the space detection model so that the mask regions are segmented in detail.

For another example, the training system may generate a saturation-adjusted training rebar image by editing a training rebar image so that its saturation is adjusted. To this end, the training system may adjust the saturation of the training rebar image by adjusting the pixel intensities for a plurality of pixels belonging to the training rebar image. Therefore, the training system may train the space detection model to more accurately distinguish between regions corresponding to rebars and mask regions in the rebar image.

With the configurations as described above, the training system may generate the plurality of training rebar images 13 in which editing corresponding to at least one of flipping, rotation, contrast adjustment, or saturation adjustment has been performed for a specific training rebar image.

Further, the training system may label a training mask region corresponding to a space between the plurality of rebars for each of the plurality of training rebar images 13 (14).

Specifically, the training system may input a bounding box corresponding to the mask region as a training mask region for each of the plurality of training rebar images and label the training mask region previously input for each training rebar image.

Therefore, the training system may train the space detection model to estimate the mask region in a rebar image using the plurality of training rebar images and the training mask regions labeled for each of the plurality of training rebar images (15).

With reference to FIG. 2, in an embodiment, the space detection model may be trained to detect the mask region in a rebar image on the basis of deep vision network (DVNet), segmentation pyramid pooling network (SPPNet), and deep CNN network (DCNet).

With reference to FIG. 3 in this regard, it may be confirmed the form in which the space detection model according to an embodiment is implemented.

With reference back to FIG. 1, the system 100 for providing a rebar arrangement state according to the present invention may classify the plurality of mask regions estimated for the rebar image into a similar group and a unsimilar group on the basis of similarity in size between the mask regions (16), and provide a rebar arrangement state according to the classification result (17).

Here, the similar group may refer to a group in which the size of spaces between a plurality of rebars is similar because the arrangement of a plurality of rebars is uniform. That is, the similar group may include a plurality of mask regions with similar sizes among a plurality of mask regions detected in the rebar image.

For example, the similar group may include a plurality of mask regions that fall within a predetermined range from an average size value of the plurality of mask regions.

For another example, the similar group may include a plurality of mask regions that fall within a predetermined range (e.g., the number of mask regions or a ratio to total mask regions) from the largest size value (or the smallest size value) with respect to an average size value of the plurality of mask regions.

The unsimilar group may refer to a group in which the arrangement of the plurality of rebars is non-uniform, resulting in different sizes of spaces between the plurality of rebars, or may refer to a group that includes the mask regions excluding the plurality of mask regions belonging to the similar group. That is, the unsimilar group may include a plurality of mask regions among the plurality of mask regions detected in the rebar image, excluding the plurality of mask regions belonging to the similar group.

With reference to FIG. 4, in an embodiment, the system 100 for providing a rebar arrangement state may classify the plurality of mask regions into a first group (e.g., A, B, and C) and a second group (e.g., P, Q, R, S, T, U, X, Y, and Z) on the basis of the number of pixels in each of the plurality of mask regions detected in the rebar image.

Subsequently, the system 100 for providing a rebar arrangement state may specify a group with more mask regions between the first group and the second group (e.g., the second group) and classify the specified group again into a first subgroup (e.g., P, Q, R, S, T, and U) and a second subgroup (e.g., X, Y, and Z).

Accordingly, the system 100 for providing a rebar arrangement state may specify the group with more mask regions between the first subgroup and the second subgroup (e.g., the first subgroup) as the similar group, and specify the other mask regions, excluding the plurality of mask regions included in the similar group, as the unsimilar group.

With reference to FIG. 5 in this regard, in an embodiment, it may be confirmed the method by which the system 100 for providing a rebar arrangement state classifies the plurality of mask regions into a similar group and a unsimilar group.

Meanwhile, with reference to FIG. 6, the system 100 for providing a rebar arrangement state according to the present invention may include an input unit 110, a storage unit 120, an output unit 130, and a controller 140.

The input unit 110 may receive the rebar image as input. To this end, the input unit 110 may be connected to a separate device, system, or server, etc., in which the rebar image is provided, through a wireless or wired network, and may receive the rebar image from the device or server, etc. Alternatively, the input unit 110 may be connected to a capturing device, such as a camera, through a wireless or wired network, and receive the rebar image captured by the capturing device.

The storage unit 120 may store data and instructions necessary for the operation of the system 100 for providing a rebar arrangement state according to the present invention. For example, the storage unit 120 may store the rebar image and information related to the rebar arrangement state generated for the rebar image (e.g., similar group and unsimilar group). In addition, the storage unit 120 may store a pre-trained space detection model.

The output unit 130 may output at least one of the rebar image or the information related to the rebar arrangement state generated for the rebar image. To this end, the output unit 130 may be connected to an output device, such as a display device, through a wireless or wired network. Therefore, the output unit 130 may output the rebar image and the information related to the rebar arrangement state so as to be visually identified by a user.

Meanwhile, the output unit 130 may also be connected to a separate device, system, or server through a wireless or wired network. In this case, the output unit 130 may transmit at least one of the rebar image or the information related to the rebar arrangement state to the device, system, or server.

The controller 140 may control the overall operation of the system 100 for providing a rebar arrangement state according to the present invention. For example, the controller 140 may input the rebar image into the space detection model to obtain a plurality of mask regions, classify the plurality of mask regions into a similar group and a unsimilar group, and output the rebar arrangement state based on at least one of the similar group or the unsimilar group.

Based on the configuration of the system 100 for providing a rebar arrangement state as described above, the following will provide a more detailed description of a method of providing a rebar arrangement state.

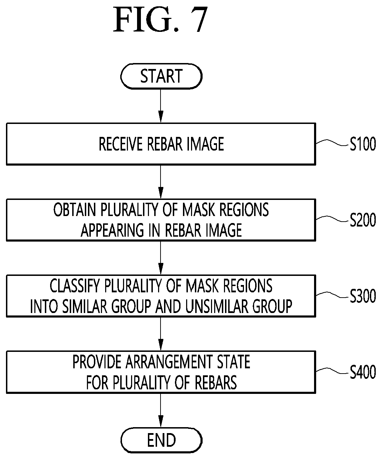

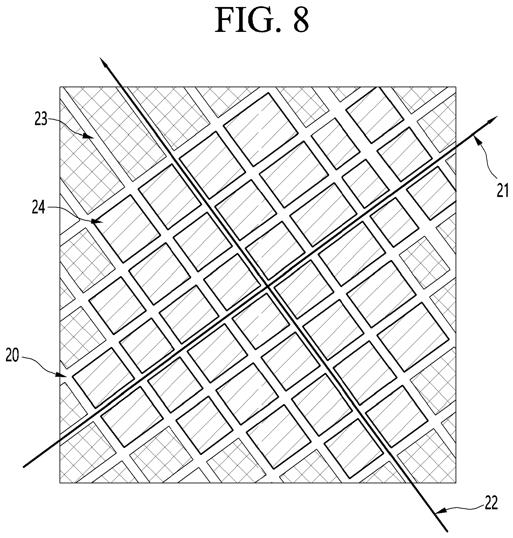

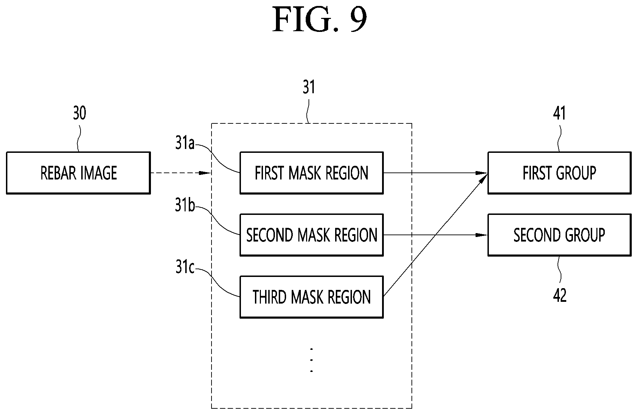

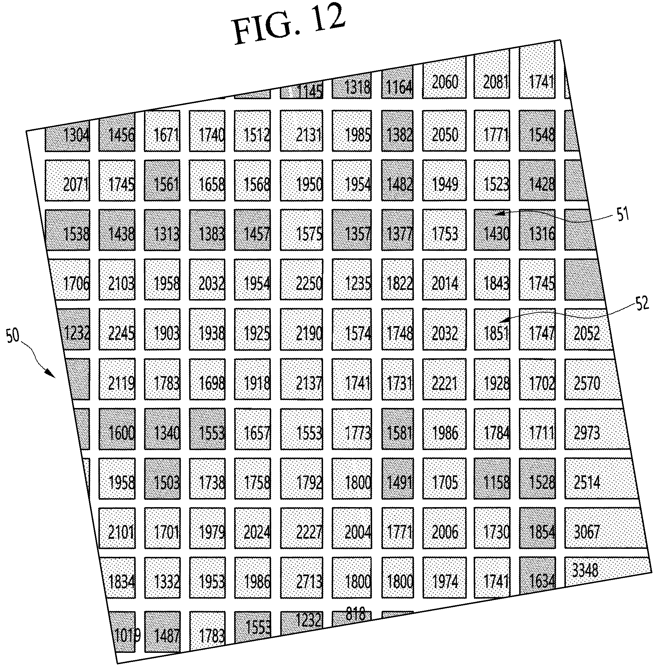

FIG. 7 is a flowchart illustrating a method of providing a rebar arrangement state according to the present invention. FIG. 8 illustrates an embodiment of detecting a mask region in a rebar image. FIGS. 9 to 11 illustrate an embodiment of classifying a mask region. FIG. 12 illustrates an embodiment of providing a rebar arrangement state.

With reference to FIG. 7, the system 100 for providing a rebar arrangement state according to the present invention may receive a rebar image captured of a space in which a plurality of rebars are arranged (S100), and input the rebar image into a pre-trained space detection model to estimate mask regions corresponding to spaces between the plurality of rebars, thereby obtaining a plurality of mask regions appearing in the rebar image (S200).

Specifically, the system 100 for providing a rebar arrangement state may receive a rebar image captured of a space in which a plurality of rebars are arranged in a grid form, and input the previously received rebar image into the pre-trained space detection model to obtain each of a plurality of regions corresponding to the spaces between a plurality of rebars formed by the plurality of rebars arranged in a grid form as a mask region.

With reference to FIG. 8, for example, the system 100 for providing a rebar arrangement state may input a rebar image into the space detection model to obtain, as a mask region 24, a rectangular space formed by two adjacent rebars among a plurality of rebars arranged along a first axis 21 and two adjacent rebars among a plurality of rebars arranged along a second axis 22 in an rebar image 20 of a grid-formed rebar arrangement 23 formed by the plurality of rebars arranged along the first axis 21 and the plurality of rebars arranged along the second axis 22.

With reference back to FIG. 7, the system 100 for providing a rebar arrangement state according to the present invention may classify the plurality of mask regions into a similar group and a unsimilar group on the basis of similarity in size between the plurality of mask regions (S300).

Specifically, the system 100 for providing a rebar arrangement state may count the number of a plurality of pixels belonging to each of the plurality of mask regions and classify the plurality of mask regions into a first group and a second group on the basis of the counted number of the plurality of pixels.

With reference to FIG. 9, for example, the system 100 for providing a rebar arrangement state may calculate the average number of pixels for a plurality of mask regions 31 on the basis of the number of the plurality of pixels belonging to each of the plurality of mask regions 31 obtained for the rebar image 30.

Accordingly, the system 100 for providing a rebar arrangement state may specify a plurality of mask regions among the plurality of mask regions 31 with the number of pixels greater than the average number of pixels (e.g., first mask region 31a and third mask region 31c, etc.) as a first group 41 (or second group 42), and specify a plurality of mask regions with the number of pixels less than the average number of pixels (e.g., second mask region 31b, etc.) as the second group 42 (or first group 41).

For another example, the system 100 for providing a rebar arrangement state may classify (or cluster) the plurality of mask regions obtained for the rebar image into a first group and a second group using the K-means clustering technique.

Further, the system 100 for providing a rebar arrangement state may count the number of the plurality of mask regions classified into the first group and the second group, specify one of groups with the largest number of mask regions that has been previously counted between the first group and the second group, and classify the plurality of mask regions into a first subgroup and a second subgroup on the basis of the number of pixels for each of the plurality of mask regions belonging to the previously specified group.

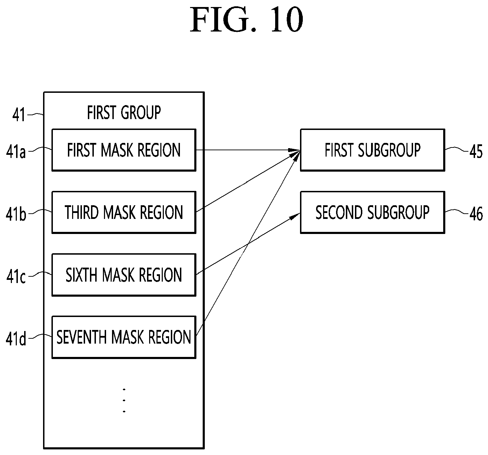

With reference to FIG. 10, for example, the system 100 for providing a rebar arrangement state may classify the plurality of mask regions belonging to the first group 41 (e.g., 41a, 41b, 41c, and 41d) into a first subgroup 45 and a second subgroup 46 when the number of mask regions included in the first group 41 is greater than the number of mask regions included in the second group, between the first group 41 and the second group 42.

Alternatively, the system 100 for providing a rebar arrangement state may classify the plurality of mask regions belonging to the second group into the first subgroup 45 and the second subgroup 46 when the number of mask regions included in the first group 41 is less than the number of mask regions included in the second group, between the first group 41 and the second group 42.

In this case, the system 100 for providing a rebar arrangement state may calculate the average number of pixels for the plurality of mask regions included in the first group 41 (or second group) on the basis of the number of pixels for each of the plurality of mask regions included in the first group 41 (or second group), and specify the plurality of mask regions among the plurality of mask regions included in the first group 41 (or second group) with the number of pixels greater than the average number of pixels (e.g., first mask region 41a, third mask region 41b, and seventh mask region 41d) as the first subgroup 45 (or second subgroup 46), and specify the plurality of mask regions with the number of pixels less than the average number of pixels (e.g., sixth mask region 41c) as the second subgroup 46 (or first subgroup 45).

Alternatively, the system 100 for providing a rebar arrangement state may classify (or cluster) the plurality of mask regions included in the first group 41 (or second group) into the first subgroup 45 and the second subgroup 46 using the K-means clustering technique.

For another example, the system 100 for providing a rebar arrangement state may specify a group to which one of mask regions with the largest (or smallest) number of previously counted pixels belongs among the plurality of mask regions classified into a first group and a second group, and classify the plurality of mask regions belonging to the specified group into a first subgroup and a second subgroup.

Further, the system 100 for providing a rebar arrangement state may count the number of the plurality of mask regions classified into the first subgroup and the second subgroup, specify one of subgroups with the largest number of the previously counted plurality of mask regions between the first subgroup and the second subgroup as the similar group, and specify the other mask regions, excluding the plurality of mask regions belonging to the similar group, among the plurality of mask regions obtained from the rebar image as the unsimilar group.

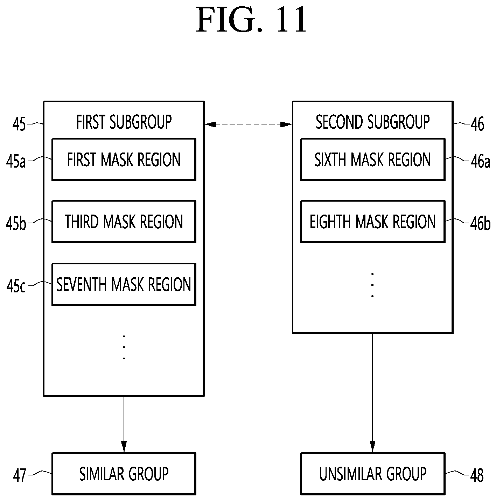

With reference to FIG. 11, for example, the system 100 for providing a rebar arrangement state may specify the first subgroup 45 (or second subgroup 46) as a similar group 47 when the number of mask regions included in the first subgroup 45 (or second subgroup 46) is greater than the number of mask regions included in the second subgroup 46 (or first subgroup 45), between the first subgroup 45 and the second subgroup 46.

In addition, the system 100 for providing a rebar arrangement state may specify one of subgroups (e.g., first subgroup 45) specified as the similar group 47 and the other subgroup (e.g., second subgroup 46) as the unsimilar group, between the first subgroup 45 and the second subgroup 46.

That is, the system 100 for providing a rebar arrangement state may specify the plurality of mask regions (e.g., first mask region 45a, third mask region 45b, and seventh mask region 45c) included in one of subgroups (e.g., first subgroup 45) with a greater number of mask regions, between the first subgroup 45 and the second subgroup 46, as the similar group 47, and specify the plurality of mask regions (e.g., sixth mask region 46a and eighth mask region 46b) included in the other subgroup (e.g., second subgroup 46) as the unsimilar group 48.

With reference back to FIG. 7, the system 100 for providing a rebar arrangement state according to the present invention may provide an arrangement state of the plurality of rebars by displaying the plurality of mask regions corresponding to at least one of the similar group or the unsimilar group on the rebar image (S400).

Specifically, the system 100 for providing a rebar arrangement state may generate a highlight to distinguish the plurality of mask regions belonging to the unsimilar group from other mask regions among the plurality of mask regions obtained from the rebar image.

With reference to FIG. 12, for example, the system 100 for providing a rebar arrangement state may, on the rebar image 50, designate and display a plurality of mask regions 51 specified as the unsimilar group with a first color (e.g., red), and designate and display a plurality of mask regions 52 specified as the similar group with a second color (e.g., green).

For another example, the system 100 for providing a rebar arrangement state may selectively display only either a plurality of mask regions specified as the unsimilar group or a plurality of mask regions specified as the similar group on the rebar image.

With the configurations as described above, the system 100 for providing a rebar arrangement state according to the present invention may efficiently verify the structural arrangement of the rebar intervals by detecting the spaces formed between rebars in the captured image of the rebar arrangement and classifying the detected spaces according to size.

To this end, the system 100 for providing a rebar arrangement state, according to the present invention can augment the training rebar image in various ways, enabling more accurate and detailed detection of the spaces formed between rebars in the rebar image.

In particular, the system and method for providing a rebar arrangement state, according to the present invention, can repeatedly perform the process of classifying the spaces formed between rebars according to size multiple times, thereby enabling more accurate detection of regions where errors have occurred in the structural arrangement of rebar intervals.

Further, the present invention described above may be implemented as a program executed by one or more processes in an electronic device and stored on a computer-readable recording medium.

Therefore, the present invention may be implemented as computer-readable code or instructions on a medium in which the program is recorded. That is, the various control methods according to the present invention may be provided in the form of a program, either in an integrated or individual manner.

Meanwhile, the computer-readable medium includes all kinds of storage devices for storing data readable by a computer system. Examples of computer-readable media include hard disk drives (HDDs), solid state disks (SSDs), silicon disk drives (SDDs), ROMs, RAMs, CD-ROMs, magnetic tapes, floppy discs, and optical data storage devices.

Further, the computer-readable medium may be a server or cloud storage that includes storage and that the electronic device is accessible through communication. In this case, the computer may download the program according to the present invention from the server or cloud storage, through wired or wireless communication.

Further, in the present invention, the computer described above is an electronic device equipped with a processor, that is, a central processing unit (CPU), and is not particularly limited to any type.

Meanwhile, it should be appreciated that the detailed description is interpreted as being illustrative in every sense, not restrictive. The scope of the present invention is defined by the appended claims.

## Claims

1. A computer-implemented method for providing a rebar arrangement state, the method comprising:
receiving a rebar image captured of a space in which a plurality of rebars are arranged;
inputting the rebar image to a pre-trained space detection model configured to estimate mask regions corresponding to spaces between the plurality of rebars, and obtaining a plurality of mask regions appearing in the rebar image;
classifying the plurality of mask regions into a similar group and a unsimilar group on the basis of similarity in size between the plurality of mask regions; and
displaying, in the rebar image, the plurality of mask regions corresponding to at least one of the similar group or the unsimilar group to provide an arrangement state of the plurality of rebars.

2. The method of claim 1, wherein the classifying of the plurality of mask regions into a similar group and a unsimilar group includes:
counting the number of a plurality of pixels belonging to each of the plurality of mask regions; and
classifying the plurality of mask regions into a first group and a second group on the basis of the counted number of the plurality of pixels.

3. The method of claim 2, wherein the classifying of the plurality of mask regions into a similar group and a unsimilar group includes:
counting the number of the plurality of mask regions classified into each of the first group and the second group;
specifying one of groups between the first group or the second group that has the largest number of the counted plurality of mask regions; and
classifying the plurality of mask regions belonging to the specified group into a first subgroup and a second subgroup on the basis of the number of pixels for each of the plurality of mask regions belonging to the specified group.

4. The method of claim 3, wherein the classifying of the plurality of mask regions into a similar group and a unsimilar group includes:
counting the number of the plurality of mask regions classified into each of the first subgroup and the second subgroup; and
specifying one of subgroups between the first subgroup or the second subgroup that has the largest number of the counted plurality of mask regions as the similar group, and specifying the other mask regions, excluding the plurality of mask regions belonging to the similar group, among the plurality of mask regions obtained from the rebar image, as the unsimilar group.

5. The method of claim 1, wherein in the obtaining of the plurality of mask regions appearing in the rebar image, each of a plurality of regions corresponding to spaces between the plurality of rebars, which are formed by the plurality of rebars arranged in a grid form, is obtained as the mask region.

6. The method of claim 1, wherein the pre-trained space detection model is trained based on a space detection model training method, the space detection model training method including:
storing a training rebar image;
generating a plurality of training rebar images by augmenting the training rebar image;
labeling training mask regions corresponding to spaces between the plurality of rebars for each of the plurality of training rebar images; and
training the space detection model to estimate the mask region in the rebar image using the plurality of training rebar images and the training mask region labeled for each of the plurality of training rebar images.

7. The method of claim 6, wherein the training rebar image is a rebar image captured by arranging a capturing device at different positions with respect to the space in which the plurality of rebars are arranged.

8. The method of claim 6, wherein in the generating of the plurality of training rebar images, for the training rebar image, the plurality of training rebar images are generated in which editing according to at least one of flipping, rotation, contrast adjustment, or saturation adjustment having been performed.

9. A system for providing a rebar arrangement state, comprising:
a storage unit storing a rebar image captured of a space in which a plurality of rebars are arranged; and
a controller configured to input the rebar image to a pre-trained space detection model configured to estimate mask regions corresponding to spaces between the plurality of rebars, and to obtain a plurality of mask regions appearing in the rebar image,
wherein the controller:
classifies the plurality of mask regions into a similar group and a unsimilar group on the basis of similarity in size between the plurality of mask regions; and
displays the plurality of mask regions corresponding to at least one of the similar group or the unsimilar group in the rebar image to provide an arrangement state of the plurality of rebars.

10. A program stored on a computer-readable recording medium, and executed by one or more processes in an electronic device, the program comprising instructions to allow the program to perform:
receiving a rebar image captured of a space in which a plurality of rebars are arranged;
inputting the rebar image to a pre-trained space detection model configured to estimate mask regions corresponding to spaces between the plurality of rebars, and obtaining a plurality of mask regions appearing in the rebar image;
classifying the plurality of mask regions into a similar group and a unsimilar group on the basis of similarity in size between the plurality of mask regions; and
displaying, in the rebar image, the plurality of mask regions corresponding to at least one of the similar group or the unsimilar group to provide an arrangement state of the plurality of rebars.

## Patentansprüche

1. Computerausgeführtes Verfahren zum Bereitstellen eines Bewehrungsanordnungszustands, wobei das Verfahren Folgendes umfasst:
Erhalten eines Bewehrungsbilds, wofür ein Raum, in dem mehrere Bewehrungen angeordnet sind, aufgenommen wurde;
Eingeben des Bewehrungsbilds in ein vortrainiertes Raumerkennungsmodell, das so eingerichtet ist, dass es Maskenbereiche, die Räumen zwischen den mehreren Bewehrungen entsprechen, schätzt, und Erhalten von mehreren Maskenbereichen, die in dem Bewehrungsbild erscheinen;
Einteilen der mehreren Maskenbereiche auf der Basis der Ähnlichkeit der Größe zwischen den mehreren Maskenbereichen in eine ähnliche Gruppe und eine unähnliche Gruppe; und
Anzeigen der mehreren Maskenbereiche, die wenigstens einer aus der ähnlichen Gruppe oder der unähnlichen Gruppe entsprechen, in dem Bewehrungsbild, um einen Anordnungszustand der mehreren Bewehrungen bereitzustellen.

2. Verfahren nach Anspruch 1, wobei das Einteilen der mehreren Maskenbereiche in eine ähnliche Gruppe und eine unähnliche Gruppe Folgendes umfasst:
Zählen der Anzahl von mehreren Pixeln, die zu jedem der mehreren Maskenbereiche gehören; und
Einteilen der mehreren Maskenbereiche auf der Basis der gezählten Anzahl der mehreren Pixel in eine erste Gruppe und eine zweite Gruppe.

3. Verfahren nach Anspruch 2, wobei das Einteilen der mehreren Maskenbereiche in eine ähnliche Gruppe und eine unähnliche Gruppe Folgendes umfasst:
Zählen der Anzahl der mehreren Maskenbereiche, die in jede aus der ersten Gruppe und der zweiten Gruppe eingeteilt wurden,
Bestimmen einer der Gruppen aus der ersten Gruppe oder der zweiten Gruppe, die die größte Anzahl der gezählten mehreren Maskenbereiche aufweist; und
Einteilen der mehreren Maskenbereiche, die zu der bestimmten Gruppe gehören, auf der Basis der Anzahl der Pixel für jeden der mehreren Maskenbereiche, die zu der bestimmten Gruppe gehören, in eine erste Untergruppe und eine zweite Untergruppe.

4. Verfahren nach Anspruch 3, wobei das Einteilen der mehreren Maskenbereiche in eine ähnliche Gruppe und eine unähnliche Gruppe Folgendes umfasst:
Zählen der Anzahl der mehreren Maskenbereiche, die in eine jede aus der ersten Untergruppe und der zweiten Untergruppe eingeteilt wurden; und
Bestimmen einer der Untergruppen aus der ersten Untergruppe oder der zweiten Untergruppe, die die größte Anzahl der gezählten mehreren Maskenbereiche aufweist, als die ähnliche Gruppe, und Bestimmen der anderen Maskenbereiche mit Ausnahme der mehreren Maskenbereiche, die zu der ähnlichen Gruppe gehören, unter den mehreren Maskenbereichen, die aus dem Bewehrungsbild erhalten wurden, als die unähnliche Gruppe.

5. Verfahren nach Anspruch 1, wobei bei dem Erhalten der mehreren Maskenbereiche, die in dem Bewehrungsbild erscheinen, jeder von mehreren Bereichen, die Räumen zwischen den mehreren Bewehrungen, die durch die mehreren in einer Gitterform angeordneten Bewehrungen gebildet werden, entsprechen, als ein Maskenbereich erhalten wird.

6. Verfahren nach Anspruch 1, wobei das vortrainierte Raumerkennungsmodell auf der Basis eines Raumerkennungsmodell-Trainingsverfahrens trainiert wird, wobei das Raumerkennungsmodell-Trainingsverfahren Folgendes umfasst:
Speichern eines Trainingsbewehrungsbilds;
Erzeugen von mehreren Trainingsbewehrungsbildern durch Erweitern des Trainingsbewehrungsbilds;
Annotieren von Trainingsmaskenbereichen, die Räumen zwischen den mehreren Bewehrungen entsprechen, für jedes der mehreren Trainingsbewehrungsbilder; und
Trainieren des Raumerkennungsmodells für das Schätzen des Maskenbereichs in dem Bewehrungsbild unter Verwendung der mehreren Trainingsbewehrungsbilder und des Trainingsmaskenbereichs, der für jedes der mehreren Trainingsbewehrungsbilder annotiert wurde.

7. Verfahren nach Anspruch 6, wobei das Trainingsbewehrungsbild ein Bewehrungsbild ist, das durch Anordnen einer Aufnahmevorrichtung an verschiedenen Positionen in Bezug auf den Raum, in dem die mehreren Bewehrungen angeordnet sind, aufgenommen wurde.

8. Verfahren nach Anspruch 6, wobei bei dem Erzeugen der mehreren Trainingsbewehrungsbilder für das Trainingsbewehrungsbild mehrere Trainingsbewehrungsbilder erzeugt werden, an denen eine Bearbeitung gemäß wenigstens einem aus einer Spiegelung, einer Drehung, einer Kontrastanpassung oder einer Sättigungsanpassung vorgenommen wurde.

9. System zum Bereitstellen eines Bewehrungsanordnungszustands, aufweisend
eine Speichereinheit, die ein Bewehrungsbild, wofür ein Raum, in dem mehrere Bewehrungen angeordnet sind, aufgenommen wurde, speichert; und
eine Steuerung, die so eingerichtet ist, dass sie das Bewehrungsbild in ein vortrainiertes Raumerkennungsmodell, das so eingerichtet ist, dass es Maskenbereiche, die Räumen zwischen den mehreren Bewehrungen entsprechen, schätzt, eingibt und mehrere Maskenbereiche, die in dem Bewehrungsbild erscheinen, erhält,
wobei die Steuerung
die mehreren Maskenbereiche auf der Basis der Ähnlichkeit der Größe zwischen den mehreren Maskenbereichen in eine ähnliche Gruppe und eine unähnliche Gruppe einteilt; und
die mehreren Maskenbereiche, die wenigstens einer aus der ähnlichen Gruppe oder der unähnlichen Gruppe entsprechen, in dem Bewehrungsbild anzeigt, um einen Anordnungszustand der mehreren Bewehrungen bereitzustellen.

10. Programm, das auf einem computerlesbaren Speichermedium gespeichert ist und durch einen oder mehrere Prozesse in einer elektronischen Vorrichtung ausgeführt wird, wobei das Programm Befehle enthält, die dem Programm gestatten,
ein Bewehrungsbild, wofür ein Raum, in dem mehrere Bewehrungen angeordnet sind, aufgenommen wurde, zu erhalten;
das Bewehrungsbild in ein vortrainiertes Raumerkennungsmodell, das so eingerichtet ist, dass es Maskenbereiche, die Räumen zwischen den mehreren Bewehrungen entsprechen, schätzt, einzugeben, und mehrere Maskenbereiche, die in dem Bewehrungsbild erscheinen, zu erhalten;
die mehreren Maskenbereiche auf der Basis der Ähnlichkeit der Größe zwischen den mehreren Maskenbereichen in eine ähnliche Gruppe und eine unähnliche Gruppe einzuteilen; und
die mehreren Maskenbereiche, die wenigstens einer aus der ähnlichen Gruppe oder der unähnlichen Gruppe entsprechen, in dem Bewehrungsbild anzuzeigen, um einen Anordnungszustand der mehreren Bewehrungen bereitzustellen.

## Revendications

1. Procédé Informatisé pour fournir un état d'agencement de barres d'armature, la méthode comprenant :
la réception d'une image de barres d'armature capturée d'un espace dans lequel une pluralité de barres d'armature sont disposées ;
l'entrée de l'image de barres d'armature dans un modèle de détection d'espace pré-entraîné configuré pour estimer les zones de masque correspondant aux espaces entre la pluralité de barres d'armature, et l'obtention d'une pluralité de zones de masque apparaissant dans l'image de barres d'armature ;
la classification de la pluralité de zones de masque en un groupe similaire et un groupe non similaire sur la base de la similarité de taille entre la pluralité de zones de masque ; et
l'affichage, dans l'image de barres d'armature, de la pluralité de zones de masque correspondant à au moins un groupe parmi le groupe similaire ou le groupe non similaire pour fournir un état d'agencement de la pluralité de barres d'armature.

2. Procédé selon la revendication 1, dans lequel la classification de la pluralité de zones de masque en un groupe similaire et un groupe non similaire comprend :
le comptage du nombre d'une pluralité de pixels appartenant à chacune de la pluralité de zones de masque ; et
la classification de la pluralité de zones de masque en un premier groupe et un second groupe sur la base du nombre compté de la pluralité de pixels.

3. Procédé selon la revendication 2, dans lequel la classification de la pluralité de zones de masque en un groupe similaire et un groupe non similaire comprend :
le comptage du nombre de zones de masque classifiées dans chacun des premier et second groupes ;
la spécification d'un des groupes entre le premier groupe ou le second groupe qui a le plus grand nombre de la pluralité comptée de zones de masque ; et
la classification de la pluralité de zones de masque appartenant au groupe spécifié en un premier sous-groupe et un second sous-groupe sur la base du nombre de pixels pour chacune de la pluralité de zones de masque appartenant au groupe spécifié.

4. Procédé selon la revendication 3, dans lequel la classification de la pluralité de zones de masque en un groupe similaire et un groupe non similaire comprend :
le comptage du nombre de zones de masque classifiées dans chacun des premier sous-groupe et second sous-groupe ; et
la spécification d'un sous-groupe parmi le premier sous-groupe ou le second sous-groupe qui a le plus grand nombre de la pluralité comptée de zones de masque comme groupe similaire, et la spécification des autres zones de masque, à l'exclusion de la pluralité de zones de masque appartenant au groupe similaire, parmi la pluralité de zones de masque obtenues à partir de l'image de barres d'armature, comme groupe non similaire.

5. Procédé selon la revendication 1, dans lequel, dans l'obtention de la pluralité de zones de masque apparaissant dans l'image de barres d'armature, chacune d'une pluralité de zones correspondant à des espaces entre la pluralité de barres d'armature, qui sont formées par la pluralité de barres d'armature agencées sous forme de grille, est obtenue en tant que zone de masque.

6. Procédé selon la revendication 1, dans lequel le modèle de détection d'espace pré-entraîné est entraîné sur la base d'un procédé d'apprentissage de modèle de détection d'espace, le procédé d'apprentissage de modèle de détection d'espace comprenant :
le stockage d'une image de barres d'armature d'apprentissage ;
la génération d'une pluralité d'images de barres d'armature d'apprentissage en augmentant l'image de barres d'armature d'apprentissage ;
l'étiquetage de zones de masque d'apprentissage correspondant à des espaces entre la pluralité de barres d'armature pour chacune de la pluralité d'images de barres d'armature d'apprentissage ; et
l'apprentissage du modèle de détection d'espace pour estimer la zone de masque dans l'image de barres d'armature au moyen de la pluralité d'images de barres d'armature d'apprentissage et de la zone de masque d'apprentissage étiquetée pour chacune de la pluralité d'images de barres d'armature d'apprentissage.

7. Procédé selon la revendication 6, dans lequel l'image de barres d'armature d'apprentissage est une image de barres d'armature capturée en disposant un dispositif de capture à des positions différentes par rapport à l'espace dans lequel la pluralité de barres d'armature sont agencées.

8. Procédé selon la revendication 6, dans lequel, lors de la génération de la pluralité d'images de barres d'armature d'apprentissage, pour l'image de barres d'armature d'apprentissage, la pluralité d'images de barres d'armature d'apprentissage sont générées, une édition selon au moins l'un parmi un retournement, une rotation, un ajustement de contraste ou un ajustement de saturation ayant été effectuée.

9. Système pour fournir un état d'agencement de barres d'armature, comprenant :
une unité de stockage stockant une image de barres d'armature capturée d'un espace dans lequel une pluralité de barres d'armature sont disposées ; et
un contrôleur configuré pour entrer l'image de barres d'armature dans un modèle de détection d'espace pré-entraîné configuré pour estimer les zones de masque correspondant aux espaces entre la pluralité de barres d'armature, et pour obtenir une pluralité de zones de masque apparaissant dans l'image de barres d'armature,
le contrôleur :
classifiant la pluralité de zones de masque en un groupe similaire et un groupe non similaire sur la base de la similarité de taille entre la pluralité de zones de masque ; et
affichant la pluralité de zones de masque correspondant à au moins un du groupe similaire ou du groupe non similaire dans l'image de barres d'armature pour fournir un état d'agencement de la pluralité de barres d'armature.

10. Programme stocké sur un support d'enregistrement lisible par ordinateur et exécuté par un ou plusieurs processus dans un dispositif électronique, le programme comprenant des instructions permettant au programme d'exécuter :
la réception d'une image de barres d'armature capturée d'un espace dans lequel une pluralité de barres d'armature sont disposées ;
l'entrée de l'image de barres d'armature dans un modèle de détection d'espace pré-entraîné configuré pour estimer les zones de masque correspondant aux espaces entre la pluralité de barres d'armature, et l'obtention d'une pluralité de zones de masque apparaissant dans l'image de barres d'armature ;
la classification de la pluralité de zones de masque en un groupe similaire et un groupe non similaire sur la base de la similarité de taille entre la pluralité de zones de masque ; et
l'affichage, dans l'image de barres d'armature, de la pluralité de zones de masque correspondant à au moins un groupe parmi le groupe similaire ou le groupe non similaire pour fournir un état d'agencement de la pluralité de barres d'armature.
